# EUROPEAN PATENT SPECIFICATION

(11) **EP 2 114 530 B1**
(45) Date of publication and mention of the grant of the patent: **21.07.2010**
(21) Application number: 08751020.2
(22) Date of filing: 28.01.2008
(51) Int. Cl.: A61K 31/4439, A61P 1/00

(54) **TREATMENT OF FAECAL INCONTINENCE**
BEHANDLUNG FÄKALISCHER INKONTINENZ
TRAITEMENT DE L'INCONTINENCE FÉCALE

(30) Priority: 26.01.2007 FR 0700548; 01.02.2007 US 898713 P
(43) Date of publication of application: 11.11.2009
(73) Proprietor: Urogene, 75010 Paris (FR)
(72) Inventor: FERTE, Jacques, F-06740 Chateauneuf (FR); BIENAYME, Hugues, F-69360 St Symphorien D'Ozon (FR); PEREZ MARTINEZ, Francisco, Carlos, E-02002 Albacete (ES)
(74) Representative: Maureau, Philippe
(86) International application number: PCT/IB2008/001296
(87) International publication number: WO 2008/090480

(56) References cited:
- EP-A2- 0 287 982
- BURGARD E C ET AL: "NEW PHARMACOLOGICAL TREATMENTS FOR URINARY INCONTINENCE AND OVERACTIVE BLADDER" CURRENT OPINION IN INVESTIGATIONAL DRUGS, PHARMAPRESS, US, vol. 6, no. 1, January 2005 (2005-01), pages 81-89, XP009067991 ISSN: 1472-4472
- HUFF ET AL: "559Development of besipirdine for treatment of Alzheimer's disease" NEUROBIOLOGY OF AGING, TARRYTOWN, NY, US, vol. 17, no. 4, 1996, page S139, XP005005593 ISSN: 0197-4580
- E. D. EHRENPREIS, D. CHANG, E. EICHENWALD: "Pharmacotherapy for Fecal Incontinence : a Review" DISEASES OF THE COLON AND RECTUM, vol. 50, 2007, pages 641-649, XP002439662

## Description

The present invention relates to the applications in gastroenterology of compounds of N-(4-pyridinyl)-1H-indol-1-amine type, and more particularly to their use in the treatment of faecal incontinence.

Faecal incontinence (also known as intestinal incontinence or anal incontinence) is a medical conditions which is defined by an inability to control defecation, or an involuntary or inappropriate loss of liquids or stools via the anus. The second International Consultation on Incontinence proposed a working definition of faecal incontinence, similar to that recommended for urinary incontinence, according to which anal incontinence is "an involuntary loss of flatulence, of liquids or of stools representing a social or hygiene problem".

In common practice, the distinction between anal incontinence and faecal incontinence is however commonly accepted, anal incontinence relating to the combined loss of gas and stools, whereas faecal incontinence is reflected by losses of stools without gas.

Moreover, although it is often excluded by the tools for evaluating faecal incontinence, urgent defecation is an additional symptom which, like urgent urination, can have a considerable influence on the quality of life of patients. Urgent defecation corresponds to a sudden need to produce stools and is generally due to a dysfunction of the external anal sphincter. While this need does not necessarily end with an episode of faecal incontinence, it may, on the other hand, constitute a precursor symptom, ignorance of which during the clinical evaluation may lead to the severity of the disease being underestimated (Vaizay et al., 1999).

Faecal incontinence (FI) affects individuals of all ages and of both sexes, and in all cases has a devastating effect on the quality of life of the individuals who suffer from this condition. The prevalence of this disease varies from 2% to 18% in adults and increases with age, particularly in institutionalized patients or patients suffering from psychiatric pathologies, and is more frequent in women.

Faecal incontinence can be attributed to many factors which affect the normal anatomy and the physiology of the anorectum. Among its known causes are, in particular, obstetric trauma, anorectal surgery, benign colorectal diseases, cancers of the pelvic region, inflammatory bowel and neurological diseases, ageing, drug treatments, food intolerances, rectal prolapse, congenital abnormalities and radiation-induced proctitis.

As disclosed in the outline of recommendations of the National Institute for Health and Clinical Excellence (NICE) on the treatment of faecal incontinence, there is no consensus on the methods of classification of the symptoms and of the causes of faecal incontinence. The most common classifications are based on:
A symptom: for example, if the patient feels an urgent need before leakage (urgent faecal incontinence) or, on the contrary, has no feeling (passive incontinence).
The nature of the leakage: for example, solid, liquid, mucous or gas.
The group of patients: for example, fragile elderly individuals, patients suffering from neurological pathologies, women with obstetric injuries.
The presumed primary cause: for example, damage to or a weakness of the internal or external anal sphincter, faecal load, sensory or motor neurological condition, cognitive deficiency, problems of access to toilets, rectal capacity, digestive motility or stool consistency.

Moreover, numerous other causes and contributing factors are possible, such as, inter alia, diet and fluid intake, drug treatments or alternatively psychological state.

The NICE working group responsible for the development of a recommendation consider that the majority of patients aged 18 and over complaining of faecal incontinence (according to the definition "involuntary loss of liquids or of solid stools") can very probably be identified as corresponding to one of the following groups:
anorectal structural anomaly (for example, trauma or degeneration of the sphincter, perianal fistula, rectal prolapse);
neurological conditions or pathologies (for example, multiple sclerosis, damage to the spinal cord, spina bifida, cerebral stroke);
constipation/faecal load (for example, diet, drug treatment, megarectum);
cognitive and/or behavioural disorders (for example, dementia, learning deficiencies);
soft stools (for example, gastrointestinal problems such as chronic inflammatory bowel diseases (CIBDs) or irritable bowel syndrome (IBS);
incontinence related to a handicap (for example, patients who are weak, suffering or have an acute or chronic handicap);
idiopathic incontinence (for example, autonomous adults with faecal incontinence and who do not correspond to any group described above).

The treatments for faecal incontinence can be distinguished according to the following groups:
conservative interventions,
medical devices,
drug treatments,
surgical procedures, in the most extreme cases.

The conservative interventions consist mainly of:
changes in lifestyle (sport, work, giving up smoking, modification to the diet and fluid intake);
measures of assistance with daily activities (adaptation of clothing, absorbent products, bags, stoppers, adaptation of the fitting out of toilets, control of odour, skincare);
intestinal control and a retraining programme (planning for going to the toilet, resisting urgent need, behaviour modification, rectal irrigation, digital stimulation or the like, abdominal massage);
functional readaptation by stimulation (and/or pelvic or sphincter exercises). This is a first choice therapy in faecal incontinence. This conditioning method, which uses visual, verbal or auditory signals, improves rectal sensation and rectoanal coordination, and brings about contraction of the external anal sphincter. The success rate of various series range from 40% to 85%, which rates could probably be predicted by the patient's motivation and the cause of the pathology rather than by the duration of faecal incontinence, manometric or endoanal ultrasound measurements or else the functional readaptation technique used.

The conservative measures are simple to implement, but are often of limited effectiveness.

Several techniques making use of medical devices exist which can benefit patients suffering from faecal incontinence. The "proton continence" device (Incontinence Control Devices, Inc., Kingwood, TX) is a flexible catheter with a photosensor and a balloon, which can be inserted into the rectum in order to send a signal warning of the arrival of stools. This device has shown an improvement in continence in certain patients. Moreover, submucosal injections of a wide variety of agents have been used to increase internal anal sphincter tone {silicone, silicone-based agents [Bioplastique ^{®}], carbon-coated beads [Acyst™ procedure], carbon-coated zirconium oxide beads [Durasphere^{®}], autologous fat, collagen crosslinked with glutaraldehyde [GAX], polytetrafluoroethylene [Polytedf]) and have shown a partial symptomatic improvement in resting anal pressure and in continence.

The Secca^{®} procedure (Curon Medical, Inc., Sunnyvale, CA) uses radiofrequency energy controlled by the temperature,of the anal canal and of the distal rectum in order to create scarring of the external anal sphincter and tissue fibrosis. In a multicentre prospective study, Efron et al. have reported an improvement in faecal incontinence and in the quality of life with disappearance of symptoms in 60% of patients. Takahashi et al. have reported similar results after a follow-up of patients for 2 years.

Another treatment for faecal incontinence is sacral nerve stimulation (SNS) which, by "neuromodulating" the somatic voluntary nerves and the afferent and efferent autonomic nerves, makes it possible to stimulate both the somatic and the autonomic innervation of the pelvic organs and of the anorectal region, through the sacral and pudendal nerves. The results published with this procedure are encouraging, with a marked improvement in continence of up to 100% and restoration of complete continence in 41% to 75% of cases.

All these procedures have a certain effectiveness, but their use is restrictive.

Drug treatments for faecal incontinence are limited to anti-diarrhoea agents and to laxatives, enemas and suppositories, which make it possible to evacuate the bowel. At low dose, amitryptiline (tricyclic antidepressant), via its anticholinergic and serotoninergic activities, could also improve continence. On the other hand, no medicament that acts on sphincter tone is currently registered specifically for the treatment of faecal incontinence.

At the current time, the only alternative in patients suffering from external sphincter disorders but without a neurological condition (intact pudendal nerves) is, after failure of conservative therapies, surgery. In these cases, obstetric lesions are the most common cause of faecal incontinence. The surgical procedure is usually an anterior sphincteroplasty, which preserves all of the fibrotic scars and overlaps the two sides of the external anal sphincter with nonabsorbable sutures. Although approximately 80% of patients initially benefit from this operation in the short term, the rate of incontinence increases, after 5 to 10 years, up to 85%.

In patients suffering from severe incontinence with significant neurological involvement, who have a multifocal lesion of the external anal sphincter that cannot be treated, or after functional failure of an anatomically successful sphincteroplasty, it could be beneficial to perform a reconstruction with neosphincter procedures using striated muscles (gracilis, gluteus maximus, sartorius, adductor longus). Transposition of the gracilis muscle is the most commonly used technique. In order to prevent fatigue and to keep contraction constant, this procedure involves the implantation of an electrical stimulator. Dynamic graciloplasty (stimulated transposition of the gracilis muscle) has brought about a significant improvement in faecal incontinence in 55 to 78% of patients. The procedure is complex, associated with a substantial morbidity (mainly infectious complications) and is no longer available in the United States.

The artificial intestinal sphincter is an inflatable silicone sleeve connected to a pump and to a balloon which regulates the pressure. The sleeve is placed around the anal canal in order to maintain the basal pressure. The pump is then implanted into the scrotum or the labia majora. It is the patient who controls the deflation of the sleeve, resulting in displacement of the fluid in the balloon located behind the pubis. A multicentre trial showed that the device was functional in 67% of patients during the first year after surgery. Despite a considerable amount of complications, such as an erosion, an infection (in 25% of cases), failure of the device leading to its removal (in 37% of cases), and re-operations (in 46% of cases), the overall outcome is positive in approximately 50% of patients.

Finally, a colostomy or an ileostomy should be envisaged only in patients suffering from severe faecal incontinence, for whom multiple procedures have failed, or who are immobilized or considered to be at high risk. This procedure may be temporary, the aim then being to protect complex reconstructions and to improve the quality of life until complete distal healing.

The present invention relates to the use of a family of compounds for obtaining a medicament for use in the treatment of faecal incontinence.

The compounds of the invention correspond to formula (I): 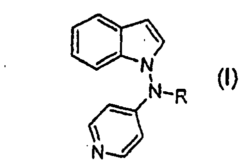 in which R represents an alkyl, alkylene, alkylidyne, cycloalkyl, cycloalkylene, cycloalkylidyne or -CONH₂ group, and also -COR' or -COOR' groups, in which R' is chosen from alkyl, alkylene, alkylidyne, cycloalkyl, cycloalkylene and cycloalkylidyne groups, it being possible for said groups R and/or R' to be substituted and/or interrupted with -O-, -COO-, -OCO-, -NHCO- and -CONH- functions.

The pharmaceutically acceptable salts of these compounds are part of the invention. It may in fact be preferable to prepare, purify and/or store a salt corresponding to the active compound, for example a pharmaceutically acceptable salt. Examples of pharmaceutically acceptable salts are given in the publication Berge et al., "Pharmaceutically acceptable salts", J. Pharm. Sci., 66, 1-19 (1977). By way of examples, mention may be made of salicylic, hydrochloric and fumaric salts.

The invention relates more particularly to the compounds of N-(4-pyridinyl)-1H-indol-1-amine type of general structure (I) when the group R is equal to a hydrogen atom, N-(4-pyridinyl)-1H-indol-1-amine (compound HP748), or to the n-propyl group, N-propyl-N-(4-pyridinyl)-1H-indol-1-amine or besipirdine (compound HP749), and also pharmaceutically acceptable salts thereof, and more particularly their use for obtaining a medicament for use in the treatment of symptoms related to faecal incontinence.

According to the invention, two abovementioned compounds can advantageously be associated or combined in a medicament. A preferred association or combination comprises a compound of formula (I) in which R represents a hydrogen atom (HP748) and a compound of formula (I) in which R represents the n-propyl group (HP749), in the knowledge that the HP748 compound can be obtained by hepatic metabolization of the HP749 compound in humans. The combination of actions is particularly suitable for obtaining a medicament for use in the treatment of symptoms related to faecal incontinence.

Still according to the invention, an abovementioned compound can advantageously be associated or combined with amitryptiline (tricyclic antidepressant) in a medicament. A preferred association or combination comprises a compound of formula (I) in which R represents the n-propyl group (HP749).

Still according to the invention, an abovementioned compound can advantageously be associated or combined with an anti-diarrhoea agent or laxative in a medicament. A preferred association or combination comprises a compound of formula (I) in which R represents the n-propyl group (HP749).

The compounds (I) of the invention can be obtained by means of a process such as that described in patent US-4 970 218. The use of besipirdine in the treatment of urinary incontinence has been investigated in the prior art (Bargard et al., Curret OP. Inv. Drugs 2005, 6(1), 81-89).

The examples hereinafter illustrate the effect of the compounds according to the invention.

### Example: Effect of the HP749 compound on the electromyographic activity of the rabbit striated anal sphincter

The effect of the HP749 compound was tested on rabbits under conditions of bladder irritation.

### Description of the protocol:

Female rabbits of the New Zealand race weighing from 2.5 to 3.5 kg were used. The bladder irritation was caused by transcontinuous bladder infusion of 0.5% acetic acid. Under these conditions, the striated anal sphincter electromyography is increased.

Throughout the experiment, the animal was placed in the supine position and anaesthetized with 2-3% of halothane.

The striated sphincter electromyography (SS-EMG) was carried out using two electrodes (30-gauge needle) placed percutaneously in the striated anal sphincter, approximately 5-10 mm from the anus (laterally). The electric signals were amplified on an ML136 preamplifier (AD Instruments, PanLab, Barcelona, Spain), filtered below 1 Hz and above 5 KHz and presented in a PowerLab window. The striated sphincter electromyography (SS-EMG) was recorded continuously during the cystometry.

Cumulative doses of the HP749 compound (0, 1, 3 and 5 mg/kg) were administered through an intravenous canula connected to the ear vein. The medicaments in saline solution were freshly prepared before each experiment and administered in a volume of 1 ml, followed by rinsing with 1 ml of physiological saline solution.

The results were expressed by the mean ± the standard error of the mixing. The electromyographic values obtained under conditions of irritation after administration of a placebo were used as control values. The effects of the HP749 compound were analysed and compared with the control values by means of a Wilcoxon test. The results obtained were considered to be significant when p<0.05.

### Results:

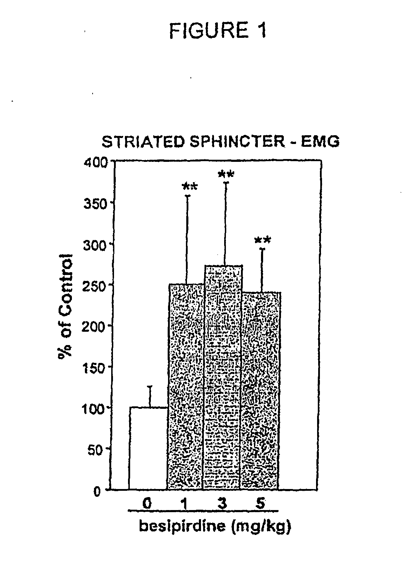

Interestingly, the HP749 compound itself showed a significant effect on the electromyographic (EMG) activity of the striated anal sphincter at all the doses (Figure 1), leading to a mean increase in the EMG activity of 155% compared with the control value (p<0.01) .

### Conclusion:

The HP749 compound showed a powerful effect on the external anal sphincter in rabbits, which effect appeared to have reached its maximum at the lowest dose tested, 1 mg/kg.

## Claims

1. Use of a compound corresponding to formula (I): 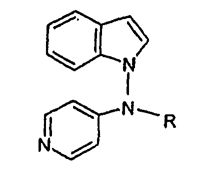 in which R represents hydrogen or a group chosen from alkyl, alkylene, alkylidyne, cycloalkyl, cycloalkylene, cycloalkylidyne and -CONH₂ groups, and the -COR' and -COOR' groups, in which R' is chosen from alkyl, alkylene, alkylidyne, cycloalkyl, cycloalkylene and cycloalkylidyne groups, it being possible for said groups R and/or R' to be substituted and/or interrupted with -O-, -COO-, -OCO-, -NHCO- or -CONH- functions, or a pharmaceutically acceptable salt of said compound, in the manufacture of a medicament for use in the treatment of faecal incontinence.

2. Use according to Claim 1, **characterized in that** R is the n-propyl group.

3. Use according to Claim 1 or 2, **characterized in that** it associates two compounds corresponding to formula (I).

4. Use according to Claim 3, **characterized in that** it associates a compound of formula (I) in which R represents H and a compound of formula (I) in which R represents the n-propyl group.

5. Use according to Claim 2, **characterized in that** it associates a compound of formula (I) in which R represents the n-propyl group and amitryptiline.

6. Use according to Claim 2, **characterized in that** it associates a compound of formula (I) in which R represents the n-propyl group and an anti-diarrhoea agent or a laxative.

## Patentansprüche

1. Verwendung einer Verbindung entsprechend der Formel (I): 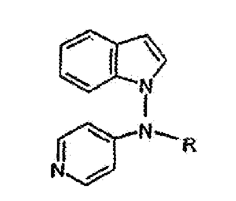 wobei R Wasserstoff oder eine Gruppe, die aus Alkyl-, Alkylen-, Alkylidin-, Cycloalkyl-, Cycloalkylen-, Cycloalkylidin- und CONH₂-Gruppen gewählt ist, und die -COR'- und -COOR'-Gruppen darstellt, wobei R' aus Alkyl-, Alkylen-, Alkylidin-, Cycloalkyl-, Cycloalkylen- und Cycloalkylidingruppen gewählt ist, wobei es für die Gruppen R und/oder R' möglich ist, durch -O-, -COO-, -OCO-, -NHCO- oder -CONH-Funktionen substituiert und/oder unterbrochen zu werden, oder eines pharmazeutisch unbedenklichen Salzes der Verbindung bei der Herstellung eines Medikaments zur Verwendung bei der Behandlung von Stuhlinkontinenz.

2. Verwendung nach Anspruch 1, **dadurch gekennzeichnet, dass** R für die n-Propylgruppe steht.

3. Verwendung nach Anspruch 1 oder 2, **dadurch gekennzeichnet, dass** sie zwei Verbindungen, die der Formel (I) entsprechen, verbindet.

4. Verwendung nach Anspruch 3, **dadurch gekennzeichnet, dass** sie eine Verbindung der Formel (I), in welcher R H darstellt, und eine Verbindung der Formel (I), in welcher R die n-Propylgruppe darstellt, verbindet.

5. Verwendung nach Anspruch 2, **dadurch gekennzeichnet, dass** sie eine Verbindung der Formel (I), in welcher R die n-Propylgruppe darstellt, und Amitryptilin verbindet.

6. Verwendung nach Anspruch 2, **dadurch gekennzeichnet, dass** sie eine Verbindung der Formel (I), in welcher R die n-Propylgruppe darstellt, und ein Antidiarrhoikum oder ein Laxans verbindet.

## Revendications

1. Utilisation d'un composé correspondant à la formule (I) : 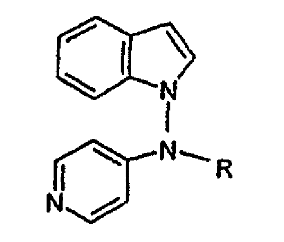 dans laquelle R représente un atome d'hydrogène ou un groupe choisi parmi les groupes alkyle, alkylène, alkylidyne, cycloalkyle, cycloalkylène, cycloalkylidyne et -CONH₂, et les groupes -COR' et -COOR', dans lesquels R' est choisi parmi les groupes alkyle, alkylène, alkylidyne, cycloalkyle, cycloalkylène, cycloalkylidyne, sachant qu'il est possible que lesdits groupes R et/ou R' soient substitués et/ou interrompus par des fonctions -O-, -COO-, - OCO-, -NHCO- ou -CONH-, ou d'un sel pharmaceutiquement acceptable du composé,
dans la fabrication d'un médicament destiné au traitement de l'incontinence fécale.

2. Utilisation selon la revendication 1, **caractérisée en ce que** R est le groupe n-propyle.

3. Utilisation selon la revendication 1 ou 2, **caractérisée en ce qu'**elle associe deux composés correspondant à la formule (I).

4. Utilisation selon la revendication 3, **caractérisée en ce qu'**elle associe un composé de formule (I) dans laquelle R représente H et un composé de formule (I) dans laquelle R représente le groupe n-propyle.

5. Utilisation selon la revendication 2, **caractérisée en ce qu'**elle associe un composé de formule (I) dans laquelle R représente le groupe n-propyle et l'amitriptyline.

6. Utilisation selon la revendication 2, **caractérisée en ce qu'**elle associe un composé de formule (I) dans laquelle R représente un groupe n-propyle et un agent antidiarrhéique ou un laxatif.
